Europäisches Patentamt

European Patent Office

Office européen des brevets

(11) Publication number: **0 351 458**
**A1**

# EUROPEAN PATENT APPLICATION

(21) Application number: **88201580.3**

(22) Date of filing: **21.07.88**

(51) Int. Cl.⁴: **C12P 21/00 , C12N 5/00 , A61K 39/395 , G01N 33/574**

(43) Date of publication of application:
**24.01.90 Bulletin 90/04**

(84) Designated Contracting States:
**AT BE CH DE ES FR GB GR LI LU NL SE**

(71) Applicant: **SCLAVO S.p.A.**
**Via Fiorentina 1**
**I-53100 Siena(IT)**

(72) Inventor: **Tecce, Mario Felice**
**Via Vico Alto 10**
**I-53100 Siena(IT)**
Inventor: **Terrana, Benedetto**
**Str. Colle Pinzuto 50**
**I-53100 Siena(IT)**

(74) Representative: **Roggero, Sergio et al**
**Ing. Barzanò & Zanardo Milano S.p.A. Via Borgonuovo 10**
**I-20121 Milano(IT)**

(54) **Process for preparing human alpha-foetoprotein.**

(57) Process and means for preparing human alpha-foetoprotein (AFP) which comprises cultivating in a serum-free medium cells of human hepatoma capable of secreting alpha-foetoprotein as the major proteinic component, and of secreting albumin in negligible amounts relatively to alpha-foetoprotein, and separating and purifying AFP under non-denaturant conditions.

The so obtained human alpha-foetoprotein is used for preparing specific monoclonal and polyclonal antibodies useful for the diagnosis of primary hepatic tumor, teratocarcinomas and foetal physiopathologies and for the management of primary hepatic carcinoma.

# PROCESS FOR PREPARING HUMAN ALPHA-FOETOPROTEIN

The present invention is concerned with a process and a means for preparing human alpha-foetoprotein, an antigen associated with tumor, particularly suitable in the diagnostic and therapeutical field.

Alpha-foetoprotein, in the following indicated as AFP, is an embryonal alpha-globulin, structurally similar to albumin.

Large amounts of AFP are present in embryonal serum, and they decrease rapidly after the birth, reaching in the healthy adults values comprised within the range of from 1 to 6 $\mu g/l$, but the concentration of AFP, in human biologic liquids, considerably increases in patients affected by teratocarcinoma, hepatopathies, primary hepatocarcinoma and in some foetal pathologic statuses.

The determination of AFP in blood, or in another biological liquid constitutes hence a basic and sensible clinic parameter for the diagnosis of the above said pathologic statuses and for the evaluation of the suitable therapy.

The immunologic methods, generally used for the determination of AFP, require an adequate amount of pure alpha-foetoprotein for preparing the specific antibodies, and for the reference standard.

At present, alpha-foetoprotein is obtained, by means of an extraction and purification process, from blood of umbilical cord, phoetal blood and ascitic liquid of patients affected by primary hepatocarcinoma.

However, said process is not completely satisfactory, in particular from the standpoint of the raw materials used.

In fact, the limited availability and the variability of said raw materials do not make it possible alpha-foetoprotein to be prepared in high amounts, and with reproducible characteristics. Furthermore, the presence, in said materials, of serum proteins, in particular albumin, in a concentration considerably higher than of AFP, requires the use of laborious, and not entirely satisfactory, purification methods. So, e.g., the techniques of purification of immunoaffinity (B. Noorgard Pedersen, Scand. J. Immunol. Suppl. 4 (1967); M.M. Baig, Anal. Biochem. (1980), 101, 200), although make it possible alpha-foetoprotein with a good purity degree to be obtained, do not allow the partially degraded or fragmented material, still capable of reacting with anti-alpha-foetoprotein antibodies, to tbe removed from the purified material. Furthermore, the same procedures of elution from the immunoaffinity columns can denature AFP present in eluate.

From the above, the need derives in the art, of having available a simple, reproducible and cheap process, as well as a means, for preparing alpha-foetoprotein, which makes it possible the above reported problems to be overcome.

Said need is met, according to the present invention, by providing a process which operates by means of human hepatoma cells, capable of secreting alpha-foetoprotein as the major proteinic component, and of secreting albumin in negligible amounts relatively to alpha-foetoprotein.

Therefore, the purpose of the present invention is a process for preparing human alpha-foetoprotein, an antigen associated with tumor, useful in the diagnostic and therapeutical field, which comprises cultivating in a liquid medium containing carbon sources, nitrogen sources, mineral salts and vitamins, which has penicillin G, streptomycin and $Na_2SeO_3$ added, free from bovine foetal serum, cells of human hepatoma capable of secreting alpha-foetoprotein as the major proteinic component, and of secreting albumin in negligible amounts relatively to alpha-foetoprotein, and separating and purifying under non-denaturant conditions the so-obtained alpha-foetoprotein.

Another purpose of the present invention is the use of the so-obtained alpha-foetoprotein for preparing specific monoclonal and polyclonal antibodies.

A further purpose of the present invention is the use of said antibodies in the therapeutical field, in order to prepare medicinal compositions against the primary hepatic tumor and in the diagnostic field for the determination of hepatopathies, primary hepatic tumor, teratocarcinomas and foetal physiopathologies.

Still another purpose of the present invention is a diagnostic kit for the determination and dosage of alpha-foetoprotein in human clinic samples, using AFP and the so-obtained antibodies.

The present invention is based on the surprising observation that human hepatic cells adapted in a culture medium containing carbon sources, nitrogen sources, mineral salts, vitamin, free from bovine foetal serum and which has penicillin G, streptomycin and $Na_2SeO_3$ added, are capable of secreting alpha-foetoprotein as the major proteinic component, and of secreting albumin in negligible amounts relatively to AFP. The acquisition, by said cells, of such a characteristic, makes it possible the process according to the present invention to be realized.

In accordance therewith, according to the present invention, the process for preparing human alpha-foetoprotein comprises:

a) cultivating in a liquid culture medium containing carbon sources, nitrogen sources, mineral salts, vitamins, free from bovine foetal serum and which has penicilling G, streptomycin and $Na_2SeO_3$ added, at approximately 37°C, cells of human hepatoma capable of secreting alpha-foetoprotein as the major proteinic component, and of secreting albumin in negligible amounts relatively to alpha-foetoprotein;

b) separating the supernatant containing raw alpha-foetoprotein from the material from cellular decay, and finally,

c) separating and purifying the raw alpha-foetoprotein from said supernatant by electrophoresis on preparation polyacrylamide gel.

Hepatic cells suitable for the process according to the present invention are human hepatoma cells.

Among these, preferred are the human hepatoma cells with a high degree of differentiation, capable of producing even minimum amounts of alpha-foetoprotein.

According to the present invention, said cells are expanded, by operating according to known general techniques, and are then adapted by cultivation in a medium free from bovine foetal serum.

In practice, the cellular expansion is carried out by cultivating the cells in minimum medium (MEM) to which bovine foetal serum (10% by weight) is added, at a temperature of 37°C, under an atmosphere containing 5% of $CO_2$, 90% humidity and 95% of air for at least two weeks.

During this time period, the culture medium is replaced, at least twice a week, by a same volume of the same MEM medium which has bovine foetal serum added, and the cells, arrived at the confluence, are separated by trypsinization with an Earle saline solution containing 0.25% of trypsin, 0.536 mM EDTA and 5 mM NaOH, at room temperature (20-25°C), for approximately 20 minutes.

The so separated cells are seeded into flasks containing the same minimum medium plus bovine foetal serum, with $5 \times 10^4$ cells being distributed per $cm^2$.

At the end of the expansion step, the cells are collected and adapted by being seeded into culture flasks, in a liquid medium containing carbon sources, nitrogen sources, mineral salts, vitamin, and which has penicillin G (50 mg/ml), streptomycin (50 mg/ml), $Na_2SeO_3$ ($3 \times 10^{-8}$ M) added, free from bovine foetal serum.

Said adaptation step is carried out at a temperature equal to, or approximately equal to, 37°C, under an atmosphere containing 5% of $CO_2$, 90% of humidity and 95% of air for at least 3 weeks.

The culture medium is periodically replaced by a same volume of the same medium, and the cells, arrived at the confluence, are separated by trypsinization with an Earle saline solution having the above reported composition, at room temperature, for approximately 3 minutes.

During this time period, the cellular adaptation is checked by determining, by means of electrophoretic analysis on polyacrylamide gel, the amounts of AFP and albumin which are secreted into the culture medium.

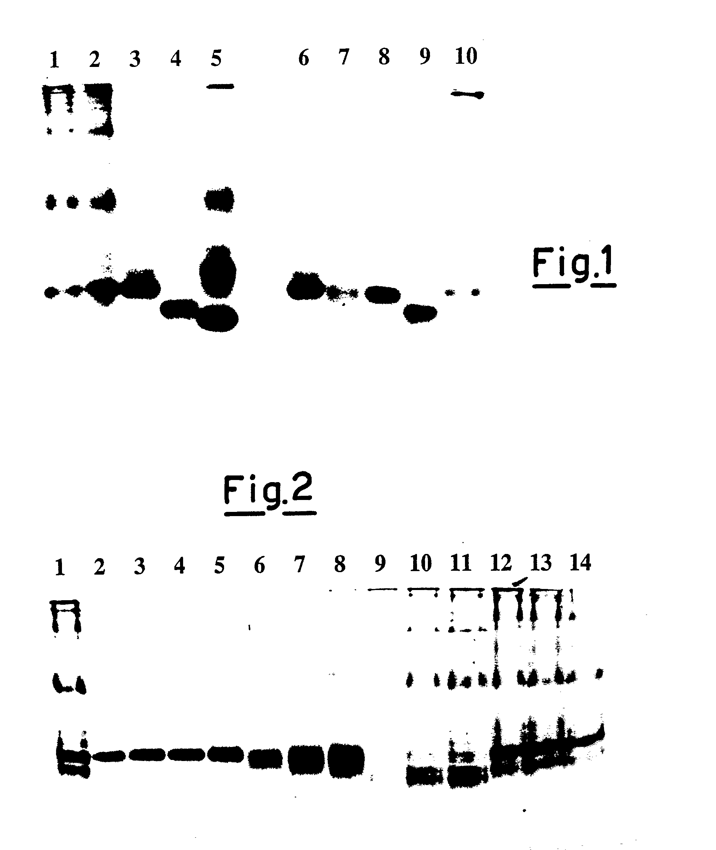

The results obtained, reported in Figures 1 and 2, show an increase of AFP from 15% to 40%, and a decrease of albumin from 40% to less than 3% of total proteins. According to the process of the present invention, the so-adapted cells are used for preparing human alpha-foetoprotein.

In practice, the cells are seeded into culture flasks in liquid medium containing carbon sources, nitrogen sources, mineral salts, vitamins, free from bovine foetal serum and which has penicillin G, streptomycin and $Na_2SeO_3$ added, and are cultivated at a temperature equal to, or approximately equal to 37°C.

The culture means is periodically collected from the individual flasks, is replaced by a same volume of the same medium, and is centrifuged in order to separate the supernatant from the material from cellular decay.

The supernatants, which contain raw alpha-foetoprotein and albumin, with the ratio of alpha-foetoprotein to albumin being comprised within the range of from 10:1 to 20:1 by weight, are combined, concentrated to an approximately 10-20 times smaller volume than the initial volume, and are purified.

The presence in the supernatant of amounts of albumin smaller than the AFP amounts, makes it possible simple purification techniques, and mild operating conditions to be used.

In accordance therewith, according to the process of the present invention, alpha-foetoprotein is purified by electrophoresis on preparative polyacrylamide gels. By operating in that way, the albumin present in the supernatant - which, due to its electrical charge, migrates differently from AFP - can be completely separated and alpha-foetoprotein in native conditions can be obtained.

The purification by electrophoresis is carried out by placing the polyacrylamide gels, whose composition is reported in Example 2, in 0.024 M tris buffer, 0.19 M glycine, pH 8.3, applying a potential difference corresponding to 10 to 15 mA, for a time comprised within the range of from 12 to 16 hours.

At the end of said time period, the alpha-foetoprotein containing gel portions are identified, are isolated and are cut into 1-2 mm cubes. Alpha-foetoprotein is then eluted, by placing said gel cubes in 50 mM tris buffer, pH 8.3, with mild stirring, at a temperature of approximately 4°C, at least twice.

The combined eluates are centrifuged twice in order to separate the acrylamide residues, are concentrated to an approximately 10-times smaller volume, and are finally freeze-dried.

Alpha-foetoprotein with a purity degree higher than 98% is thus obtained. At Western blot analysis, with anti-human albumin antibodies, the so obtained alpha-foetoprotein results in fact to be free from albumin. On the basis of the prior art, it was not foreseeable that the problem of the preparation of alpha-foetoprotein could be solved by such a simple process as the process of the present invention.

In fact, it was not foreseeable that hepatoma cells could produce alpha-foetoprotein as the major proteinic component, and, simultaneously, secrete negligible amounts of albumin.

The use of cells having these properties unknown to date, makes the process of the present invention possible for the first time.

Said process shows, as compared to the prior state of the art, considerable advantages, among which simpleness of execution, a high yield and reproducibility of the alpha-foetoprotein obtained.

This latter has to be attributed, according to the invention, to the fact that the process operates with only one, constant, AFP source.

Therefore, the alpha-foetoprotein obtained according to the process of the present invention is particularly suitable for the international standardization of diagnostic kits, and for preparing monoclonal and polyclonal antibodies obtainable by means of known general techniques, useful in the diagnostic and therapeutical field.

Short description of the Figure.

## Figure 1

The results are reported, which were obtained on an analytic polyacrylamide gel (PAGE), wherein:

In 1: the supernatant obtained from hepatoma cells at adaptation beginning, shows the presence of two bands corresponding to AFP and albumin.

In 2: the supernatant obtained from adapted cells shows the band corresponding to AFP only.

In 3, 6: AFP purified by ourselves.

In 5: the supernatant obtained from non-adapted cells shows the presence, besides AFP and albumin, of other serum proteins.

In 7 and 8: commercial AFP standard.

In 4 and 9: albumin standards (Sigma).

In 10: non-purified supernatant obtained from adapted cells.

## Figure 2

The results are reported, which were obtained on analytic polyacrylamide gels (PAGE), wherein:

In 1: the supernatant obtained from cells at adaptation beginning shows the bands corresponding to AFP and albumin.

In 2, 3, 4 and 5: increasing amounts of pure AFP obtained from adapted cells show the absence of albumin.

In 6, 7 and 8: increasing amounts of commercial AFP show, due to the width of the band, a certain microheterogeneity in preparation.

In 10 and 11: the supernatant from non-adapted hepatic cells coming from cultures on microcarrier shows a band corresponding to albumin.

In 12, 13 and 14: the supernatants obtained from hepatic cells during the adaptation step show the gradual disappearance of the band of albumin.

The following experimental examples are illustrative and non-limitative of the same invention.

## Example 1

## Adaptation of the Hepatic Cells

Hepatic cells Hep G2 (Knowles et al. Science (1980) 209, pages 497-499) are inoculated into four culture flasks of 75 cm² containing 10 ml of minimum medium M.E.M. (Eacle H. 1959 - Science, vol. 130 pages 432-437) with 10% of bovine foetal serum and incubated, at 37°C, under an atmosphere containing 5% of $CO_2$, 95% of air and with a 90% humidity, for approximately 2 weeks.

The medium is replaced twice a week, and the cells, reached the confluence, are detached by trypsinization at 37°C for 20 minutes, with 5 ml of Earle saline solution (W.R. Earle J. Natl. Cancer Inst. 1943 page 165 Vol. 4) containing 0.25% of trypsin, 0.536 mM of EDTA and 5 mM of NaOH.

The so separated cells are seeded again into four flasks of 175 cm², containing 25 ml of the same culture medium, with 50,000 cells per cm² being distributed.

By operating in that way, at the end of the second week four flasks of 175 cm² containing the hepatic cells are obtained.

The cells are then detached by trypsinization, by operating under the same conditions as above reported, and are seeded into 8 flasks of 175 cm² containing 25 ml of R.P.M.I. 1640 medium (GIBCO), having $3 \times 10^{-8}$ M of $Na_2SeO_3$, 50 mg/ml of penicillin G and 50 mg/ml of streptomycin added, and free from bovine foetal serum, with 50,000 cells/cm² being distributed.

The flasks are maintained at 37°C, under the same conditions as above reported, for at least 3 weeks, with the culture medium being replaced by an equal volume of the same medium twice a week.

The cells, reached the confluence, are detached by trypsinization, at room temperature, for 3 minutes, and are seeded again into other flasks containing the same R.P.M.I. 1640 medium.

At the end of the second week, the hepatic cells result perfectly adapted, i.e., capable of producing and secreting into the culture medium high amounts of alpha-foetoprotein and of secreting negligible albumin amounts as compared to AFP.

The changes in alpha-foetoprotein and albumin concentrations produced during the various adaptation steps were determined by non-denaturant electrophoresis, in the absence of sodium dodecylsulphate (S.D.S.) according to Laemli (Nature 1970, vol. 227, pages 680-685) and the results obtained are shown in Figures 1 and 2.

AFP and albumin in the supernatant respectively vary form 15 to 40% and from 40% to less than 3% of total proteins.

<div align="center">Example 2</div>

<div align="center">Preparation of Human-Foetoprotein from Adapted Hepatoma Cells</div>

a) HepG2 cells, adapted as reported in Example 1, are seeded into 100 flasks of 175 cm², containing 30 ml of R.P.M.I. 1640 medium (GIBCO), having $3 \times 10^{-8}$ M of $Na_2SeO_3$, 50 mg/ml of penicillin G and 50 mg/ml of streptomycin added.
The flasks are incubated at 37° C, under an atmosphere at 5% of $CO_2$, 95% of air and 90% humidity for 45 days during which the culture medium is periodically collected, centrifuged in order to remove the material from cellular decay and replaced by the same medium. The supernatants are combined and stored at -30° C.
By operating in that way, during the first 15 days 20 litres of supernatant were collected, which contained 43 mg/l of raw alpha-foetoprotein and approximately 2 mg/l of albumin.

b) Purification of alpha-foetoprotein

Five litres of supernatant, containing a total amount of 215 mg of raw AFP, obtained as reported in above (a) step, is concentrated to an end volume of approximately 400 ml, using the Minitan system by Millipore, using slabs with an exclusion power of up to 10,000 daltons. The concentrate is then charged, as aliquots of 12 ml, on preparative polyacrylamide gel (PAGE), in the absence of denaturant agents.

The preparative gels, of dimensions of 170x135x4.5 mm, are constituted by:
10% acrylamide;
0.266% bis-acrylamide;
0.374 M tris pH 8.3;
0.05% ammonium persulphate;
0.083% TEMED (N,N,N′,N′-tetramethylethylenediamine).

On these gels, a gel of 25x130x4.5 mm is then superimposed, which has the following composition: 3.75% acrylamide;
0.1% bis-acrylamide;
0.125 M tris pH 6.8;
0.05% ammonium persulphate;
0.083 TEMED.

The gels are run at 15 mA/gel for 16 hours, in 0.0247 M tris buffer, 0.19 M glycine, pH 8.3.

At the end of the electrophoretic run, the gels are removed from the buffer, two sides stripes of approximately 1 cm and a central stripe of approximately 0.3 cm are cut, and the residual gel is stored at -20° C. After colouring with Comassie blue the alpha-foetoprotein in the stripes, these latter are realigned with the residual gel, so that the AFP-containing gel portions can be identified.

These latter portions are subsequently separated, cut into cubes of 1-2 mm, and are eluted twice in 6 ml/gel stripe of 50 mM tris buffer, pH 8.3, with mild stirring, for a total time of 20 hours.

The elution liquid, containing AFP as its major proteinic component, is centrifuged twice, in order to separate the residues of acrylamide, is concentrated to an approximately 10 times higher concentration with Millipore Cx30 depressure filters, and is freeze-dried.

In that way, from 1 litre of supernatant, 19 mg of alpha-foetoprotein with a purity of 98-99% is obtained (yield 45%).

At western blot analysis with anti-human albumin antibodies, the obtained alpha-foetoprotein results to be free from albumin.

In Figure 2, the results are reported, which are obtained by means of preparative analytical gel (PAGE), by comparing different amounts of alpha-foetoprotein purified as above reported (2, 3, 4 and 5) to human commercial alpha-foetoprotein (6, 7, 8).

For this latter, the microheterogeneity has to be noticed.

Claims

1. Cells of human hepatoma capable of secreting alpha-foetoprotein as the major proteinic component, and of secreting albumin in a negligible amount relatively to alpha-foetoprotein.

2. Cells of human hepatoma according to claim 1, characterized in that secreted alpha-foetoprotein and albumin show a mutual ratio comprised within the range of from 10:1 to 20:1 by weight.

3. Cells of human hepatoma according to claims 1 and 2, obtained by adapting cells of human hepatoma in a liquid culture medium containing carbon sources, nitrogen sources, mineral salts, and vitamins, which has penicillin G, streptomycin and $Na_2SeO_3$ added, free from bovine foetal serum, at a temperature equal to, or approximately equal to, 37° C for the necessary time in order to obtain alpha-foetoprotein and albumin in the mutual ratio of from 10:1 to 20:1.

4. Process for preparing human alpha-foetoprotein characterized in that:

a) the cells of human hepatoma as defined in claims from 1 to 3 are cultivated in a liquid culture medium containing carbon sources, nitrogen sources, mineral salts, vitamins, free from bovine foetal serum and which has penicilling G, streptomycin and $Na_2SeO_3$ added, at a temperature of approximately 37° C;

b) the material from cellular decay is separated from the culturing medium and the supernatant containing raw alpha-foetoprotein as the major proteinic component is recovered, and, finally,

c) the raw alpha-foetoprotein is separated and purified from the so obtained supernatant by electrophoresis on preparative polyacrylamide gel.

5. Process according to claim 4, wherein in the (a) step the culture medium is R.P.M.I. 1640.

6. Process according to claim 4, wherein in the (b) step the supernatant is separated by centrifugation at 3,000 rpm for 10 minutes.

7. Process according to claim 4, wherein in the (c) step the electrophoresis is carried out in tris buffer at pH 8,3, at 20-25° C, at a current comprised within the range of from 10 to 15 mA/gel, for a time comprised within the range of from 12 to 16 hours.

8. Use of the alpha-foetoprotein obtained as reported in claims from 4 to 7, for preparing specific monoclonal and polyclonal antibodies.

9. Use of the antibodies according to claim 8, for preparing therapeutical compositions for the treatment of human primary hepatic carcinoma.

10. Use of the antibodies according to claim 8, for the diagnosis of pathologic affections, which cause an increase in alpha-foetoprotein in human body liquids.

11. Diagnostic kit for the determination and dosage of alpha-foetoprotein in biological human samples, characterized in that it uses the alpha-foetoprotein and the specific monoclonal and polyclonal antibodies obtained as defined in claims from 4 to 8.

1   2   3   4   5       6   7   8   9   10

_Fig.1_

_Fig.2_

1   2   3   4   5   6   7   8   9   10  11  12  13  14

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 108, no. 15, 11th April 1988, page 404, abstract no. 128039k, Columbus, Ohio, US; M.F. TECCE et al.: "High-yield and high-degree purification of human alpha-fetoprotein produced by adaptation of the human hepatoma cell line HEP G2 in a serum-free medium", & ANAL. BIOCHEM. 1988, 169(2), 306-11 * Abstract * | 1-11 | C 12 P 21/00 C 12 N 5/00 A 61 K 39/395 G 01 N 33/574 |
| X | CHEMICAL ABSTRACTS, vol. 108, no. 1, 4th January 1988, page 392, abstract no. 4013c, Columbus, Ohio, US; G.J. DARLINGTON et al.: "Growth and hepatospecific gene expression of human hepatoma cells in a defined medium", & IN VITRO CELL. DEV. BIOL. 1987, 23(5), 349-54 * Abstract * | 1-11 | |
| X | CHEMICAL ABSTRACTS, vol. 97, no. 15, 11th October 1982, page 344, abstract no. 123411v, Columbus, Ohio, US; H. NAKABAYASHI et al.: "Growth of human hepatoma cell lines with differentiated functions in chemically defined medium", & CANCER RES. 1982, 42(9), 3858-63 * Abstract * | 1-7 | TECHNICAL FIELDS SEARCHED (Int. Cl.4) C 12 P |

The present search report has been drawn up for all claims

| Place of search | Date of completion of the search | Examiner |
|---|---|---|
| THE HAGUE | 21-03-1989 | REMPP G.L.E. |